Europäisches Patentamt

(19) European Patent Office                (11) Veröffentlichungsnummer : **0 041 189**

Office européen des brevets                                    **B1**

(12)                    **EUROPÄISCHE PATENTSCHRIFT**

(45) Veröffentlichungstag der Patentschrift :        (51) Int. Cl.³ : **C 12 P 21/00, A 61 K 45/02**
   22.06.83

(21) Anmeldenummer : 81103918.9

(22) Anmeldetag : 21.05.81

(54) Verfahren zur Herstellung von Interferon II und stimulierte Klone zur Durchführung des Verfahrens.

(30) Priorität : 22.05.80 DE 3019621
            11.02.81 DE 3104900

(43) Veröffentlichungstag der Anmeldung :
   09.12.81 Patentblatt 81/49

(45) Bekanntmachung des Hinweises auf die Patenterteilung : 22.06.83 Patentblatt 83/25

(84) Benannte Vertragsstaaten :
   AT BE CH DE FR GB IT LI LU NL SE

(56) Entgegenhaltungen :
   FR A 2 203 622
   FR A 2 257 306
   CHEMICAL ABSTRACTS, Band 67, Heft 15,
   9. Oktober 1967, Selte 6764, Zusammenfassung
   71985u, COLUMBUS, OHIO (US) R. M. FRIEDMAN
   et al. : « Stimulation of interferon production in
   human lymphocytes by mitogens »
   ARCH. INTERN MED., Band 126, Juli 1970, CHI-
   CAGO (US) E. DE CLERCQ et AL. : « Induction of
   Interferon by Nonviral Agents, Seiten 94-108
   THE JOURNAL OF IMMUNOLOGY, Band 112, Heft
   2, Februar 1974, BALTIMORE (US) L. B. EPSTEIN
   et ai. : « Evaluation of T Lymphocyte Effector
   Function in Immunodeficiency Diseases : Abnormality in Mitogen-Stimulated Interferon in
   Patients with Selective IgA Deficiency »
   NATURE, Band 280, Heft 5724, August 1979,
   BASINGSTOKE (US) J. M. ZARLING et al. :
   « Continuous culture of T cells cytotoxic for autologous human leukaemia cells », Seiten 685-688
   NATURE, Band 283, Heft 5745, Januar 1980,
   BASINGSTOKE (US) P. NEWMARK : « Engineered E. Coll produce Interferon », Seite 323

(73) Patentinhaber : **Stiftung Deutsches Krebsforschungszentrum**
   **Im Neuenheimer Feld 280**
   **D-6900 Heidelberg 1 (DE)**

(72) Erfinder : **Krammer, Peter, Dr.**
   **Bunsenstrasse 19a**
   **D-6900 Heidelberg (DE)**
   Erfinder : **Kirchner, Holger, Prof.Dr.**
   **Häusserstrasse 33**
   **D-6900 Heidelberg (DE)**
   Erfinder : **Marcucci, Fabrizio, Dr.**
   **Unterer Burggarten 19**
   **D-6901 Dossenheim (DE)**

(74) Vertreter : **Patentanwälte Müller-Boré, Deufel,**
   **Schön, Hertel, Lewald, Otto**
   **Postfach 86 07 20 Siebertstrasse 4**
   **D-8000 München 86 (DE)**

### Verfahren zur Herstellung von Interferon II und stimulierte Klone zur Durchführung des Verfahrens

Erfindungsgegenstand ist ein Verfahren zur Herstellung von Typ II Interferon aus T-Lymphozyten durch Sensibilisierung und Selektionierung von Klonen aus heterogenen T-Lymphozyten-Populationen, dadurch gekennzeichnet, dass diese Zellklone einer mitogenen Stimulation unterworfen werden und nach Weiterzüchtung der stimulierten Klone Typ II Interferon, insbesondere aus den zellfreien Überständen der Kulturen, geerntet wird.

Weiterer Gegenstand sind Typ II Interferon produzierende Zellklone zur Durchführung dieses Verfahrens die aus T-Lymphozyten durch Sensibilisierung und Selektionierung von Klonen aus heterogenen T-Lymphozyten-Populationen erhalten sind, dadurch gekennzeichnet, dass diese Zellklone danach einer mitogenen Stimulation unterworfen sind.

Interferon wird von körpereigenen Zellen produziert und hat in der letzten Zeit erhöhte Aufmerksamkeit gewonnen, da es die Neusynthese von Viren hemmt. Es wird auch diskutiert, ob Interferon nicht nur bei Virus- sondern auch bei Tumorerkrankungen eine therapeutische Wirkung erzielt, und es gibt Gründe, dies zu bejahen.

Anhand biologischer und biochemischer Parameter unterscheidet man zwei Typen von Interferon, Typ I und Typ II. Typ I wird in Zellen unter anderem nach Virusinfektionen produziert, z. B. aus Leukozyten und Fibroblasten sowie Lymphoblasten (Kirchner et al., Pharmacie in unserer Zeit, 8, 113, 1979). Es gibt Grund zur Annahme, dass Typ II Interferon aus Immunozyten erhältlich ist (Kirchner et al., Immunobiology 156, 65, 1979 und Kirchner et al., Eur. J. Immunol. 10 : 224, 1980).

In Tiermodellen gibt es Hinweise dafür, dass Typ II Interferon gegen Tumoren erheblich wirksamer ist als Typ I Interferon.

Obwohl die erste Arbeit über Interferon 1957 veröffentlicht wurde, ist die Herstellung immer noch ein Problem. Eine wesentliche Voraussetzung für den experimentellen und klinischen Einsatz von Interferon jedoch ist seine Reindarstellung basierend auf der Produktion in ausreichender Quantität. Dafür gibt es bisher für Typ II Interferon keine zufriedenstellende Möglichkeit.

Der Erfindungsgegenstand liefert nun ein Verfahren zur Herstellung von Typ II Interferon in grossem Masstab und gestattet die Erzielung von erhöhten Konzentrationen an Typ II Interferon in den bei der Herstellung anfallenden Lösungen.

Das nun gefundene Verfahren und die stimulierten Klone können die Basis für eine industrielle Grossproduktion von Typ II Interferon darstellen.

Wichtig bei diesem Verfahren ist, dass es sich im Gegensatz zu verschiedenen bei Typ I angewandten Verfahren bei den Interferon produzierenden Zellen um normale Zellen und nicht um Tumorzellen handelt, wobei die gestellte Aufgabe im Wesentlichen dadurch gelöst wird, dass Klone aus heterogenen Populationen selektioniert und die ausgewählten Klone mit mitogenen Stimulantien nachstimuliert werden.

Es ist überraschend, dass man hier einen Erfolg erzielt, da man annahm, dass diese Zellklone nicht mehr stimulierbar wären.

Gemäss einer bevorzugten Ausführungsform werden aus den Einzelklonen homogene Massenkulturen gezüchtet und diese T-Zellklone einer mitogenen Stimulation unterworfen.

Gemäss weiteren bevorzugten Ausführungsformen erfolgt die mitogene Stimulation der T-Zellklone mit verschiedenen T-Zellmitogenen oder es werden verschiedene Konzentrationen des mitogenen Stimulationsmittels angewandt.

Nach einer weiteren bevorzugten Ausführungsform werden die nach der Stimulation erhaltenen Zellklone separat weitergezüchtet.

Nach einer weiteren bevorzugten Ausführungsform erfolgt die Stimulation der Zellklone in serumfreiem Medium.

Es ist auch bevorzugt, das zur Interferon-Produktion benötigte genetische Material der selektionierten Klone in an sich bekannter Weise in Bakterien oder Tumorzellen zur Gewinnung von Interferon zu benutzen.

T-Lymphozyten (Thymus abh. Lymphozyten) aus lymphoiden Organen (Lymphknoten oder Milz) oder dem Blut werden in Gewebekultur sensibilisiert. Diese Sensibilisierung kann z. B. durch Antigene oder T-Zellmitogene, wie Concanavalin A oder Phytohämagglutinin erfolgen. Als günstig hat sich vor allem Concanavalin A in der Konzentration von 5 µg/ml bei einer Aussaat von $5 \times 10^6$ Lymphknotenzellen/ml (4 ml Kulturen) erwiesen.

Die sensibilisierten T-Lymphozyten werden nach einer 2- bis 3-Tage dauernden Kultur gereinigt und mit Wachstumsfaktoren versetzt. Diese Wachstumsfaktoren werden aus dem Kulturüberstand von z. B. mit 5 µg Concanavalin A/ml sensibilisierten Rattenmilzlymphozyten ($5 \times 10^6$ Zellen/ml, 50 ml Kulturen) gewonnen. Dieses Verfahren führt dazu, dass einige T-Lymphozyten als normale Zellen kontinuierlich zu wachsen beginnen. Das kontinuierliche Wachstum der T-Lymphozyten ist abhängig von der Präsenz und der Menge der Wachstumsfaktoren in der Kultur. Solche T-Lymphozytenzellinien werden dann in Mikrotiterplatten unter Zugabe von bestrahlten Fütterzellen (z. B. Peritonealexudatzellen der Maus) kloniert. Aus den Einzelklonen werden homogene Massenkulturen als Nachkommen von Einzelzellen erhalten, die in grösseren Flaschen weiterkultiviert werden können und kontinuierlich wachsen. Diese Klone repräsentieren ein Spektrum von funktionell heterogenen normalen T-Lymphozyten in permanenter Gewebekultur.

Bisher wurden solche T-Zellklone entweder funktionell inaktiv gefunden, oder sie behielten die primär induzierte spezifische Aktivität, die z.

B. durch Antigenstimulation induziert worden war, bei. Es war nicht bekannt, dass die T-Zellklone durch weitere mitogene Stimulation zur Expression von funktionellen Aktivitäten gebracht werden konnten. Insbesondere sind Versuche negativ verlaufen, die zum Ziel hatten, in diesen T-Zellklonen durch Inkubation mit Mitogenen ein verstärkt proliferatives Wachstum zu erzeugen. Deswegen ist die Idee, durch Mitogenstimulation der T-Zellklone Interferon Typ II zu produzieren, nicht naheliegend sondern eine wesentliche Neuerung.

Es ist erfindungsgemäss gelungen, durch weitere Stimulation solcher Klone mit verschiedenen T-Zellmitogenen die herauszuselektionieren, die hohe Titer von Typ II Interferon produzieren. Dabei ist einmal von Bedeutung, dass klonierte Zellen in der Lage sind, Interferon Typ II zu sezernieren, zum anderen übertrifft die Menge des sezernierten Typ II Interferons die Quantität, die aus einer normalen Lymphozytenpopulation bereitgestellt wird, um ein Vielfaches. Dieses neue Verfahren erlaubt unabhängig von Speziesschranken a) die Definition von Typ II Interferon produzierenden Zellen und b) die Anreicherung von grossen Mengen von Zellen, die Typ II Interferon produzieren als Basis für die biochemische Reindarstellung für einen weiteren experimentellen und therapeutischen Einsatz.

Dieses Verfahren gestattet es, hohe Mengen an Interferon vom Typ II herzustellen ; zusätzlich kann die Konzentration an Typ II Interferon in den gewonnenen Lösungen auf mehr als das 10-fache gegenüber Massenkulturen von gemischten Zellen erhöht werden, was nicht nur für die gewonnene absolute Menge sondern auch eine Reindarstellung oder Gewinnung von konzentrierten Präparaten von Bedeutung ist.

Die folgenden Beispiele erläutern die Erfindung :

Beispiel 1

AKR/J Mäuse wurden, wie beschrieben, Krammer et al., J. Exp. Med. 151 : 1166, 1980, mit 2 × 10 µl 30 % Trinitrochlorbenzol in Aceton auf der Abdominalhaut sensibilisiert. 5 Tage nach der Sensibilisierung wurden die Lymphozyten der drainierenden axillären und inguinalen Lymphknoten in Kultur gebracht (5 × 10⁶ Zellen/ml ; 4 ml Kulturen ; Falconflaschen No. 3 013, aufrecht) und für 4 bis 5 Tage in einem 95 % Luft-5 % $CO_2$ Gemisch inkubiert. Dabei kommt es ohne Antigenrestimulierung zu einer T-Zellproliferation mit maximaler Aktivität am Tag 4 bis 5.

Die überlebenden Zellen wurden dann wie beschrieben (P.H. Krammer, J. Exp. Med. 147 : 25, 1978) durch eine Ficolldichtezentrifugation (Ficoll-Hypaque 1 077 g/cm³) gereinigt und kloniert. Bei der Klonierung wurde im statischen Mittel wie beschrieben, (Eichmann et al., J. Exp. Med. 152 : 477, 1980) eine Zelle/Loch von 96 Loch-Platten (Falcon No. 3 040) auf einem Rasen von 5 × 10⁴ Fütterzellen (Peritonealexudatzellen der AKR/J Maus) ausgesät. Die Klonierung erfolgte in Gegenwart von 10 % T-Zellwachstumsfaktorhaltigem Vollmedium.

Der Wachstumsfaktorhaltige Überstand wurde gewonnen von Rattenmilzlymphozyten, stimuliert in Gewebekultur für 24 Stunden mit Concanavalin A (5 × 10⁶/ml ; 50 ml ; 5 µg Con A/ml ; Falconflaschen N° 3 024 F, liegend) (Eichmann et al., J. Exp. Med. 152 : 477, 1980).

Die Zellen in den 96-Loch-Platten wurden 2 mal/Woche mit frischem Medium gefüttert. Nach ca. 3 Wochen zeigten sich makroskopisch sichtbare T-Zellklone, die in grossen Flaschen (Falcon N° 3 024 F) in demselben Medium weitergezüchtet wurden.

Jeweils 6 × 10⁵ Zellen in 0,2 ml Medium der einzelnen Klone werden dann nach Waschen in die Löcher einer 96-Loch-Platte gebracht und mit verschiedenen Konzentrationen von Concanavalin A stimuliert. 24 Stunden später werden die zellfreien Überstände dieser Kulturen geerntet und in üblicher Weise auf Interferonaktivität getestet (Maus L Zellen, Vesikular Stomatitis Virus, Test der Hemmung der Virusneubildung). Es ist so gelungen, Klone von T Lymphozyten zu finden, die bis zu 10 000 IE/ml von Interferon produzieren. Nach akzeptierten physiokochemischen Kriterien handelte es sich hierbei um Typ II Interferon.

Nach dieser Arbeitsweise ist es möglich, aus diesen Klonen Typ II Interferon in grosser Menge zu erzeugen.

Beispiel 2

Dieses Beispiel ist ein Anwendungsbeispiel beim Menschen.

Aus dem peripheren Blut wurden Lymphozyten durch eine Ficolldichtezentrifugation getrennt. Die somit gewonnenen Zellen wurden mit Phythämagglutinin (PHA, 1 %) aktiviert, nach 2 Tagen Kultur über Ficoll gereinigt und dann kloniert, indem im statistischen Mittel 1 bis 50 Zellen/Loch von 96-Loch-Platten (Falcon Nr. 3 040) auf einem Rasen von 2 × 10⁴ bestrahlten Fütterzellen (Mausperitonealexudatzellen) und menschlichen peripheren Blutzellen (3 × 10⁴) ausgesät wurde. Die Klonierung erfolgte in Gegenwart von 20 % T-Zellwachstumsfaktor und 1 % PHA-haltigem Vollmedium. Der Wachstumfaktor-haltige Überstand wurde gewonnen von menschlichen Lymphozyten stimuliert in Gewebekultur für 24 Stunden mit Phytohemagglutinin (5 × 10⁶/ml ; 50 ml ; 1 % PHA/ml ; Falconflaschen Nr. 3 024 F, liegend). Die Zellen in den 96-Loch-Platten wurden 1 mal/Woche mit frischem Medium und Fütterzellen gefüttert. Nach ca. 3 Wochen zeigten sich makroskopisch sichtbare T-Zellklone.

Zellen der einzelnen Klone in 0,2 ml wachstumsfaktorfreiem Medium wurden dann mit 20 µg/ml PHA stimuliert, z. B. gemäss Beispiel 1. Dieser Schritt kann, und zwar auch bei Anwendung auf menschliche Zellen, auch in serumfreiem Medium, z. B. RPMI 1 640 durchgeführt werden. 24 Stunden später wurden die zellfreien Überstände dieser Kulturen geerntet und in üblicher Weise auf Interferonaktivität getestet. Die Kulturen mit hohem Interferontiter wurden dann in Wachstumsfaktor- und PHA-haltigem Vollmedium unter Anwesenheit von bestrahlten menschlichen Fütterzellen zu Makrokulturen hochgezüchtet.

Es ergaben sich Interferontiter von mehreren hundert IE/ml.

Die grosse Menge an Typ II Interferon produzierenden Zellen gestattet es aber auch, die Interferon liefernden Gene bzw. das zur Interferonproduktion benutzte genetische Material der selektionierten Klone in an sich bekannter Weise in Bakterien oder Tumorzellen (in Gewebekultur) zur Gewinnung von Interferon zu benutzen, was ein zusätzliches Verfahren zur grosstechnischen Produktion von Type II Interferon ermöglicht. Nachdem die Isolierung von Genen und der Einbau in Bakterien bzw. Tumorzellen bzw. die Verwendung von genetischem Material zur entsprechenden Veränderung von Bakterien oder Zellen als solche bekannt sind (erstere Arbeitsweise ist schon für Typ I Interferon erfolgreich versucht worden) bedarf diese spezielle Ausführungsform keinerlei weiterer Erläuterung.

Bei der Nachstimulierung hat sich beim Arbeiten in serumfreiem Medium, bei menschlichen Zellen, eine Bevorzugung für Phytohemagglutinin (PHA) ergeben, jedoch ist z. B. auch Concanavalin A brauchbar.

Als Konzentrationen bei der Stimulation der Klone hat sich wie bei der bekannten Sensibilisierung (= Stimulation der T-Lymphozyten) zur Herstellung von Klonen eine Menge von 5 bis 30 µg/ml, insbesondere 15 bis 25, vorzugsweise 10 bis 20 µg/ml als zweckmässig erwiesen.

Der Begriff Klone ist selbstverständlich in breitem Umfang zu verstehen und umfasst auch das Arbeiten mit einer sogenannten Linie, die ja als bekannter spezifischer Sonderfall der Selektionierung von Klonen betrachtet werden kann.

Zur Terminologie, die hier benutzt wird, sei folgendes ausgeführt :

Die Behandlung der Zellen mit Stimulationsmitteln, wie Antigenen oder T-Zellmitogenen zur Herstellung der als Ausgangsmaterial verwendeten selektionierten Klone wird hier meist Sensibilisierung genannt, während die weitere Behandlung der selektionierten Zellklone mit mitogenen Stimulantien hier Stimulation genannt wird. Es kann sich natürlich in beiden Fällen um die Behandlung mit den gleichen Stimulationsmitteln unter den gleichen Bedingungen handeln, z. B. Concanavalin A oder Phytohemagglutinin. Es soll durch die unterschiedliche Bezeichnung nur die Stufe gemäss der Erfindung (Stimulation) von der bekannten Vorstufe (Sensibilisierung) unterschieden werden.

## Ansprüche

1. Verfahren zur Herstellung von Typ II Interferon aus T-Lymphozyten durch Sensibilisierung und Selektionierung von Klonen aus heterogenen T-Lymphozyten-Populationen, dadurch gekennzeichnet, dass diese Zellklone einer mitogenen Stimulation unterworfen werden und nach Weiterzüchtung der stimulierten Klone Typ II Interferon, insbesondere aus den zellfreien Überständen der Kulturen, geerntet wird.

2. Verfahren nach Anspruch 1, dadurch gekennzeichnet, dass aus den Einzelklonen homogene Massenkulturen gezüchtet und diese T-Zellklone einer mitogenen Stimulation unterworfen werden.

3. Verfahren nach einem der Ansprüche 1 oder 2, dadurch gekennzeichnet, dass die mitogene Stimulation der T-Zellklone mit verschiedenen T-Zellmitogenen erfolgt.

4. Verfahren nach Anspruch 1 bis 3, dadurch gekennzeichnet, dass verschiedene Konzentrationen des mitogenen Stimulationsmittels angewandt werden.

5. Verfahren nach Anspruch 3 oder 4, dadurch gekennzeichnet, dass die nach der Stimulation erhaltenen Zellklone separat weitergezüchtet werden.

6. Verfahren nach einem oder mehreren der vorhergehenden Ansprüche 1 bis 5, dadurch gekennzeichnet, dass die Stimulation in serumfreiem Medium durchgeführt wird, z. B. RPMI Medium.

7. Verfahren nach einem oder mehreren der vorhergehenden Ansprüche, dadurch gekennzeichnet, dass das zur Interferon-Produktion benötigte genetische Material der selektionierten Klone in an sich bekannter Weise in Bakterien oder Tumorzellen zur Gewinnung von Interferon benutzt wird.

8. Typ II Interferon produzierende Zellklone zur Durchführung des Verfahrens nach Anspruch 1 bis 7, die aus T-Lymphozyten durch Sensibilisierung und Selektionierung von Klonen aus heterogenen T-Lymphozyten Populationen erhalten sind, dadurch gekennzeichnet, dass diese Zellklone danach einer mitogenen Stimulation unterworfen sind.

## Claims

1. Process for the preparation of type II interferon from T lymphocytes by sensitising and selecting clones from heterogeneous T lymphocyte populations, characterised in that these cell clones are subjected to mitogenic stimulation and, after further culturing of the stimulated clones, type II interferon is harvested, in particular from the cell-free supernatants of the cultures.

2. Process according to Claim 1, characterised in that homogeneous mass-production cultures are produced from the individual clones and these T cell clones are subjected to mitogenic stimulation.

3. Process according to one of Claims 1 or 2, characterised in that the mitogenic stimulation of the T cell clones is carried out with various T cell mitogens.

4. Process according to Claim 1 to 3, characterised in that various concentrations of the mitogenic stimulating agent are used.

5. Process according to Claim 3 or 4, characterised in that the cell clones obtained after stimulation are further cultured separately.

6. Process according to one or more of the foregoing Claims 1 to 5, characterised in that the stimulation is carried out in a serum-free medium, for example an RPMI medium.

7. Process according to one or more of the foregoing claims, characterised in that the genetic material in the selected clones which is necessary for production of interferon is utilised in a manner known per se in bacteria or tumour cells to obtain interferon.

8. Cell clones producing type II interferon for carrying out the process according to Claims 1 to 7, which are obtained from T lymphocytes by sensitising and selecting clones from heterogeneous T lymphocyte populations, characterised in that these cell clones are thereafter subjected to mitogenic stimulation.

**Revendications**

1. Procédé de préparation de l'Interféron type II à partir de lymphocytes T par sensibilisation et sélection de clones de populations de lymphocytes T hétérogènes, caractérisé en ce que ces clones cellulaires sont soumis à une stimulation mitogène et, après culture ultérieure des clones stimulés, l'Interféron type II, en particulier provenant des résidus survivants sans cellule des cultures, est récolté.

2. Procédé selon la revendication 1, caractérisé en ce qu'à partir des clones élémentaires sont cultivées des cultures de masse et en ce que ces clones cellulaires T sont soumis à une stimulation mitogène.

3. Procédé selon une des revendications 1 ou 2, caractérisé en ce que la stimulation mitogène des clones cellulaires T s'effectue avec différents mitogènes cellulaires T.

4. Procédé selon la revendication 1 à 3, caractérisé en ce que sont utilisées différentes concentrations de l'agent de stimulation mitogène.

5. Procédé selon la revendication 3 ou 4, caractérisé en ce que les clones cellulaires obtenus après la stimulation sont soumis séparément à une culture ultérieure.

6. Procédé selon une ou plusieurs des revendications précédentes 1 à 5, caractérisé en ce que la stimulation est effectuée en milieu sans sérum, par exemple milieu RPMI.

7. Procédé selon une ou plusieurs des revendications précédentes, caractérisé en ce que le matériau génétique des clones sélectionnés nécessaire à la production d'Interféron est utilisé de façon connue en soi dans des bactéries ou cellules tumorales pour obtenir l'Interféron.

8. Clones producteurs d'Interféron type II utilisés pour la mise en œuvre du procédé selon revendications 1 à 7 qui sont obtenus à partir de lymphocytes T par sensibilisation et sélection de clones de populations de lymphocytes T hétérogènes, caractérisés en ce que ces clones cellulaires sont ensuite soumis à une stimulation mitogène.